# EUROPEAN PATENT SPECIFICATION

(11) **EP 2 394 583 B1**
(45) Date of publication and mention of the grant of the patent: **10.04.2013**
(21) Application number: 10005942.7
(22) Date of filing: 09.06.2010
(51) Int. Cl.: A61B 17/02, F16B 23/00

(54) **Surgical instrument**
Chirurgisches Instrument
Instrument chirurgical

(43) Date of publication of application: 14.12.2011
(73) Proprietor: Karl Storz GmbH & Co. KG, 78532 Tuttlingen (DE)
(72) Inventor: Thouèment, Yann, 78690 Les essarts le roi (FR); Besse, Régis, 78280 Guyancourt (FR)
(74) Representative: Fugmann, Winfried

(56) References cited:
- EP-A1- 0 926 362
- WO-A1-97/40752
- DE-U1- 29 719 063
- FR-E- 80 508
- GB-A- 1 396 518
- GB-A- 2 092 253
- US-A- 4 419 029
- US-A1- 2006 052 671

## Description

The present invention relates to a surgical instrument, comprising a screw for fastening one part of a surgical instrument to another part of the instrument. The present invention also relates to a surgical instrument set.

In some types of surgical procedures, surgical instruments are employed consisting of a multiplicity of parts which need to be fastened with respect to each other. Such instruments are assembled before a surgical intervention is conducted or during a surgical procedure, in order to adapt the instrument to requirements arising during the operation. Frequently, fastening of parts or members with respect to each other is accomplished by means of fixation screws, which provide a safe and firm connection of parts to be connected. For simplicity and ease of use, such screws usually can be turned by hand, such as thumbscrews or knurled screws.

Surgical instruments of this kind are, e. g., surgical retractors employed in heart, thorax or spinal surgery for spreading tissue in order to provide access to body structures situated below the tissue to be spread. In particular, in many heart, thorax and spinal surgeries, the sternum is divided longitudinally; the two parts of the sternum are then spread from each other, thus opening up the thorax in order to enable the surgeon to perform surgery inside or through the chest cavity. A surgical retractor usually comprises an extendable frame on which a variety of functional elements can be mounted. In particular, two arms of the extendable frame are equipped with one or several holding elements comprising blades for engaging tissue and thus holding tissue away from the surgical site. Further functional elements to be added may comprise heart holding or illumination means, or other accessories. Holding elements as well as further functional elements are equipped with screws for mounting and fixing the respective element on the frame.

As disclosed in WO 03/065901 A1, a heart holder can be mounted to a surgical retractor. In order to fix the heart holder to the frame of the retractor, a fixation screw is provided. The heart holder comprises two rods which can be swiveled with respect to each other and locked in a desired position by means of another screw. Both screws exhibit handles for easy turning by hand.

A surgical retractor system for heart surgery is marketed by Karl Storz GmbH & Co. KG (Tuttlingen, Germany) under no. 49100 with accessories (see ENDOWORLD CARDIO-VAS 3-1-D/05-2007). Among other accessories, an OPCAB stabilizer and a frame extension can be fixed to the arms of the retractor by means of fixation screws. For easy operation by hand, the fixation screws are knurled screws.

Document WO 97/4075 discloses a surgical instrument according to the preamble of claim 1.

Depending on the surgical situation and on the particular instrument employed, however, it may present some difficulty to the surgeon to operate the fixation screws by hand. Such a situation may arise, e. g., due to the instrument being placed on the body surface when turning the screws would interfere with the tissue retracted. In particular, when the retractor has a relatively small size or a multiplicity of functional elements is placed on the frame, there may be not enough space for gripping the screws firmly by hand and turning the screws. Moreover, it may not always be easy or even possible to tighten a screw with sufficient force for rigid clamping or to turn it with sufficient accuracy for exact adjustment. Providing a larger handle or wheel for turning by hand may not always be compatible with spatial limitations due to the arrangement of instruments or the surgical situation.

It is therefore an object of the present invention to provide a surgical instrument with a screw for mounting one member of the instrument upon another member and/or for fixing one member with respect to another member of the instrument, the screw being easily operable in a variety of surgical situations and/or types of surgical procedures.

This object is met by a surgical instrument according to claim 1. Particular embodiments of the surgical instrument are indicated in claims 2-5.

It is a further object of the present invention to provide a set of surgical instruments which permits flexible application and easy operation in a variety of surgical situations and/or types of surgical procedures.

This object is met by a surgical instrument set according to claim 6. Particular embodiments of the surgical instrument set are indicated in claims 7 and 8.

According to the invention, the screw of the surgical instrument comprises a tip on a first end of the screw, a thread section, and a head section adjacent to a second end of the screw. The screw has a screw axis about which it is to be turned in operation. At least the thread section may be generally cylindrical in shape, the axis of the cylinder coinciding with the screw axis. The thread may, in particular, be configured with a pitch to provide for a sufficient force when tightening the screw and/or to provide for a sufficient path length with a given number of turns. The tip of the screw may be designed flat, concave or convex, and/or may be configured to be connected to an intermediate member for improving force transfer. The thread section may be directly adjacent to the tip or may be separated from the tip by an unthreaded section, e. g., a cylinder. The thread section is configured to be inserted into a threaded section of a surgical instrument or of a part of a surgical instrument, to be screwed into or out of the threaded section of the instrument, and may be tightened against a counter-force exerted by another or the same part of the instrument against the tip of the screw.

Adjacent to the second end opposing the tip section, the screw comprises a head section. In particular, the head section may itself form a second end of the screw. The head section comprises handle means for turning the screw by hand. In general, the head section is not introduced into the threaded section of the instrument and thus remains accessible by hand. The screw may comprise further sections, such as unthreaded sections between the tip and the thread section, between the thread section and the head section, or between the head section and the second end.

The screw further comprises coupling means arranged at the second end for coupling with a drive section of a tool. In particular, the coupling means are arranged in or on the head section of the screw. Thus, the screw may be turned by hand as well as by a tool coupled to the coupling means of the screw.

Therefore, the screw configured according to the present invention can be operated by hand in any situation when this is preferable, e. g. when spatial requirements are convenient for hand operation and the required torque can be easily applied by hand-operation. On the other hand, the screw can be turned by means of a tool when this is preferable according to the surgical situation, e. g. if access by a tool is easier than by hand and/or a higher torque needs to be exerted upon the screw. Moreover, the screw can be more easily handled and inserted into the threaded section of the instrument by gripping the handle means by hand.

The screw, in particular, is employed as a fastening or fixation screw. In particular, the screw is configured for mounting or holding one member or part of a surgical instrument upon another member or part of the instrument and/or for fixing one member or part with respect to another member or part of the instrument. Thus, the screw is employed for screwing one part of a surgical instrument to another part of the instrument, for clamping or locking a movable member of a surgical instrument so that it is immovable with respect to the instrument, or for holding a part of a surgical instrument, so that the part may remain movable with respect to the instrument, but cannot be separated from it.

In particular, the screw permits a provisional mounting or fastening of one part to another part of a surgical instrument by turning the screw by hand, exerting a torque which is sufficient to turn the screw into an intermediate position in which the part is held although it may not yet be rigidly fixed or locked. This has the advantage that it is often much easier to work without a tool, which would require an extra hand for operating the tool or which would require the instrument parts to be held with only one hand. If the screw is to be tightened in order to firmly fasten or clamp the respective part of the instrument, or if access to the screw is easier accomplished with a tool, the tool is coupled to the screw and the screw turned by means of the tool. In this way, a higher torque can be applied to the screw. Thus, a most flexible and convenient use of the screw is achieved, which may be therefore employed in a variety of surgical instruments, situations, and procedures.

According to a preferred embodiment, the coupling means comprise a first axial bore. The first bore, which is substantially co-axial with the screw axis, may be a through-hole penetrating the screw along its axis, or, preferably, a dead-end bore arranged at the second end or, especially, within the handle means. A dead-end bore exhibits a bottom situated inside the screw. The bore may be, in particular, be configured for coupling with the drive section of the tool. In this way, a safe and simple coupling with the tool can be achieved.

According to a further preferred embodiment, the first axial bore has a bottom, in which a second axial bore is provided. In particular, the second bore is co-axial with the first bore, having a cross-section that is smaller than that of the first bore. The second bore may be a through-hole or a dead-end hole. The second bore may be, e. g., of cylindrical shape, with a circular cross-section, and the first bore may be generally cylindrical with a non-circular cross-section for engaging with the drive section of the tool for transmitting torque on the screw, or vice versa. In this case, that bore with the circular cross-section serves for guiding a section of the tool into the respective other bore, while that bore with the non-circular cross-section serves for turning the screw. Thus the screw permits a particular safe and simple coupling with the tool and an effective torque transfer upon the screw.

In particular, the non-circular cross-section may be hexagonal. Thus, the screw is configured for coupling with the drive section of a hexagonal screwdriver, which is also called a hex key, Allen key®, or Inbus® key. Such a configuration provides for a most simple coupling and for exerting a large torque upon the screw. Moreover, due to the obtuse angles of 120° formed by the side walls of the hexagonal bore, there are no niches where dirt or blood can remain during the procedure and stick firmly to the surface. Therefore, cleaning and sterilization is made easier and safer. For the same reason, the bottom of the bore or the bottoms of the bores can have rounded transition sections to the side walls of the respective bore.

Alternatively, the non-circular cross-section may be configured according to any other screw drive type, such as double hex, square, triple square, torx, slotted or cross-head screw drive. In particular, this pertains also if there is only one bore, or if the second bore is the one with the non-circular cross-section.

According to further embodiments, both first and second bores have non-circular cross-sections for transmitting torque. In particular, both bores may be configured according to the same screw drive type, e. g. both bores may have hexagonal cross-sections for coupling with corresponding drive sections of one or several hexagonal screwdrivers. Alternatively, both bores may be configured according to different screw drive types. In particular, the first bore may have a hexagonal cross-section, and the second bore may be configured according to a slotted or cross-head screw drive. Substantially any combination of screw drive types is possible permitting insertion through the first bore of a drive section of a screw driver corresponding to the second bore configuration.

In this way, a large variety of tools can be employed for turning the screw. Moreover, the maximal torque that can be transferred to the screw can be limited by providing a particular screw driver. If the same kind of screws is to be employed for fixing holding elements or other accessories on an arm of a surgical retractor and for operating a spreading mechanism of the retractor, e. g., the permissible torque to be applied may be much less in the latter case than in the former. A specialized tool configured for the latter case may comprise a small-sized hex, slotted, crosshead or other drive for coupling with the second bore correspondingly configured, while a tool adapted for the former case may comprise a hex drive of larger size for transferring a larger torque by coupling with a corresponding larger cross-section of the first bore. Thus, different torque limits applicable in different situations can be taken account of by configuring both bores for being driven by corresponding dif ferent tools.

In one embodiment of the present invention, the handle means are of a substantially circular cross-section. The handle means, in particular, may be configured as a wheel with a diameter enlarged with respect to the thread section of the screw, in order to facilitate gripping and turning by hand. Alternatively, the handle means may be of a substantially rectangular, in particular of a quadratic cross-section, making the handle being gripped firmly to exert a large torque on the screw. Further alternatively, the handle means may be of a substantially triangular cross-section for gripping the handle most securely and exerting a maximal torque upon the screw.

Generally, the handle means can be designed ergonomically for convenient holding and turning the screw. In particular, the shape and size of the handle means may be suited to a particular surgical instrument or procedure, as well as to the size of the surgeon's hand.

According to a further preferred embodiment, the handle means of the screw has a structured surface. This permits the screw to be more safely grasped and turned by hand, in particular with the gloves used in surgical procedures. In particular, the handle means may have a knurled surface, the handle means being designed as a knurled wheel.

According to a further preferred embodiment, the structured surface comprises ridges and grooves, the ridges and grooves extending in a direction substantially parallel to the screw axis. A structured surface of this kind can be easily manufactured and provides for a safe grip and for transmitting a considerable torque on the screw even if the surface is slippery due to contact with blood or other fluids.

In particular, the surface of the handle means of the screw may be formed by alternating concave and convex structures, which may form the ridges and grooves. The radii of curvature of the concave structures and/or the angles formed by the concave structures preferably are chosen such that blood or dirt can easily be removed for cleaning and sterilization. The radii of curvature of the convex structures and/or the angles formed by the convex structures preferable are chosen such that no sharp edges are formed, such that the surgical gloves with which the handle may be gripped are not cut or otherwise damaged.

For improved cleaning and sterilization, the surfaces of the screw, in particular the gripping surface of the handle means, may be coated with a non-stick material. Preferable, the screw is manufactured of a material that is suitable for sterilization, such as stainless steel. The thread may be configured for easy cleaning, e.g. by avoiding acute-angled hollow structures.

The present invention relates to a surgical instrument comprising a first and a second member, the first member being mountable upon and/or fixable with respect to a second member of the surgical instrument by means of a screw as described above. In particular, the surgical instrument comprises a threaded section, e. g. a threaded bore, which is configured so that the screw can be screwed into the threaded section, for fastening by screwing one part of the instrument to another part of the instrument, for clamping or locking a movable part of the instrument, or for holding a part of a surgical instrument. The thread section is configured to be inserted into the threaded section of the surgical instrument or of a part of a surgical instrument, to be turned within the threaded instrument section and possibly tightened under increased torque.

Preferably, the surgical instrument is configured for easy cleaning and sterilization after use. In particular, it is preferable that the surgical instrument can be disassembled for cleaning and sterilization. Most preferably, the screw can be detached for cleaning and sterilization. The instrument, which may be, e. g., a surgical retractor, preferably also is bio-compatible.

According to a preferred embodiment, the surgical instrument comprises a third member movable with respect to the first and/or second members by means of a drive mechanism, wherein the drive mechanism can be operated by turning a shaft, the shaft of the drive mechanism comprising a head section, the head section comprising handle means for turning the shaft by hand and coupling means for coupling with the drive section of the tool. The handle means can be similar in size and shape to the handle means of the screw described above. For ease of manufacturing and use, it is preferred that the handle means of the shaft are designed equal to the handle means of the screw. The coupling means may be similar in size and design to the coupling means of the screw described above. Preferably, the coupling means of the shaft are configured to be coupled with the same tool that can be employed for turning the screw. In this case, one tool may be sufficient for operating the drive mechanism and the screw or screws of the instrument.

According to a further preferred embodiment, the surgical instrument comprises a fourth member mountable and/or fixable directly or indirectly to the first or second member by a further screw, the further screw comprising coupling means for coupling with the drive section of the tool. The further screw may or may not comprise handle means for turning the screw by hand. The coupling means may be equal to those of the screw, so that the same tool employed for driving the screw can be coupled with the same drive section to the further screw for driving the further screw. However, the screw might comprise coupling means for engaging a first drive section of the tool in the first bore, while the further screw comprises coupling means for engaging a second drive section of the tool. In this case, one tool can be employed for operating different screws, which is of particular advantage when the further screw is at a narrow place where the above-described screw cannot be employed.

The present invention also relates to a surgical instrument set comprising a surgical instrument as described above and a tool, the tool comprising a drive section for coupling with the coupling means of the screw and a tool handle for operating the tool. Preferably, the tool is a screwdriver suitable for surgical use. In particular, the screwdriver may be a hexagonal screwdriver (hex key, Allen key®, Inbus® key). However, in principle, other kinds of screw drivers may be employed as well.

In a particularly preferred embodiment, the screwdriver comprises two drive sections, both with a hexagonal cross-section, with a first drive section having a larger diameter than a second drive section and the second drive section being situated adjacent to the first drive section and adjacent to a distal end of the screwdriver. In this embodiment, the screw of the surgical instrument comprises two bores with the first bore matching the first drive section and the second bore having a cross-section larger than that of the second drive section. In particular, the diameter of the second bore is larger than a maximal cross-sectional dimension of the second drive section. The further screw comprises only one bore matching the second drive section of the screwdriver. When inserted into the first and second bores of the screw, the screwdriver thus matches with the first bore to transfer torque and to permit driving the screw. When inserted into a bore of the further screw, the second drive section engages with the bore to permit turning the screw. This configuration permits most flexible and easy handling.

Alternatively, both drive sections may be configured according to different screw drive types for engaging with correspondingly configured first and second bores of the screw. Thus, e. g., the first drive section may be hexagonal, and the second drive section may be of a slotted or cross-head type.

The set of surgical instruments may comprise a set of screws with handle means of a variety of shapes and/or sizes. In this way, it is possible to provide handle means suitable for a variety of surgical situation or procedure, as well as handle means suiting the size of the surgeon's hands.

The features of the invention as mentioned above and as described below apply not only in the combinations mentioned but also in other combinations or alone, without leaving the scope of the present invention, in accordance with the appended claims.

Further aspects of the present invention will be apparent from the figures and from the description of a particular embodiment that follows.
Fig. 1 shows a perspective view of an exemplary surgical instrument according to the present invention;
Figs. 2a and b show a sectional and an axial view of an exemplary screw according to the present invention, respectively;
Fig. 3 shows a sectional side view of the exemplary screw placed in the exemplary instrument;
Fig. 4 shows a partial sectional view of a further screw employed in the exemplary instrument;
Fig. 5a and b show an exemplary screwdriver according to the present invention in perspective (Fig. 5a) and sectional view (Fig. 5b), inserted into the exemplary screw;
Figs. 6a and b show perspective views of two variations of exemplary screws with differently shaped handle means.

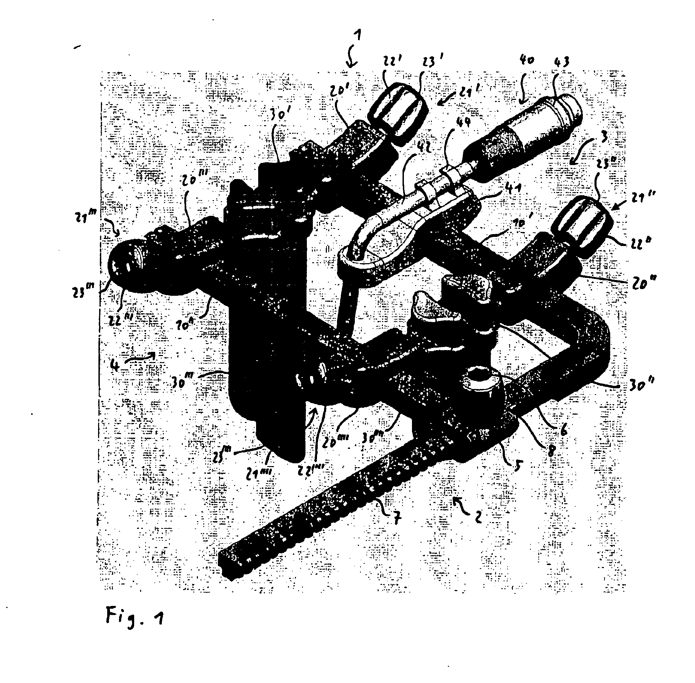

As shown in Fig. 1, an embodiment of a surgical instrument, which is a retractor 1, comprises a rail 2, a first arm 3 and a second arm 4. The first arm 3 may be integral with the rail 2 or connected to the rail 2 in a rigid manner. The second arm 4 is connected to the rail 2 by a block 5 housing a drive mechanism (not shown). The drive mechanism engages with a toothed edge 7 of the rail 2 and can be operated by turning a hand wheel 8. The hand wheel 8 exhibits a bore 6 of hexagonal cross-section for coupling with a tool. The distance between the first and second arms 3, 4 can thus be adjusted by turning the drive wheel 8. In particular, the retractor 1 can be spread or contracted by turning the drive wheel 8 in one or the other direction. Preferably, the drive mechanism is configured in a self-blocking manner, i. e., the second arm 4 can be moved only by turning the drive wheel 8, but not by an external force acting upon the second arm 4 itself.

The first arm 3 and the second arm 4 both protrude approximately at right angles from the rail, both arms being substantially parallel to each other. The first and second arms 3, 4 comprise rods 10', 10", respectively. The second arm 4 may be detachable from the rail 2. After detaching the second arm 4 from the rail, the drive mechanism can be disassembled for easy cleaning.

As is further shown in Fig. 1, two holding elements 20', 20" are mounted on the first arm 3, and two further holding elements 20"', 20"" are mounted on the second arm 4. Each of the holding elements 20', 20", 20"', 20"" holds a respective retractor blade 30', 30", 30"', 30"". In a surgical operation, the retractor 1 is placed on a body surface, the blades 30', 30", 30"', 30"" protruding into a cavity, to engage with the tissue edges to extend and hold the cavity open. On each holding element 20', 20", 20'"', 20"", a screw 21', 21 ", 21 "', 21 "" is provided with a hand wheel 22', 22", 22"', 22"" and a bore 23', 23", 23"', 23"". Each holding element 20', 20", 20"', 20"" is configured as an open frame comprising a recess into which the rod 10', 10" can be inserted and clamped within the holding element 20', 20", 20"', 20"" by the screw 21', 21", 21"', 21"".

The first arm 3 is further equipped with an illumination device 40. The illumination device 40 comprises a light guide holder 41, a light guide 42, and a light connector 43. Light guide 42 and light connector 43 form a substantially rigid unit which can be fixed on the light guide holder 41 by a snap-in mechanism 44. The light guide holder 41 comprises a recess into which the rod 10' is inserted to mount the light guide holder 41 on the arm 3. A light cable transmitting light from an external light source (not shown) can be connected to the light connector 43.

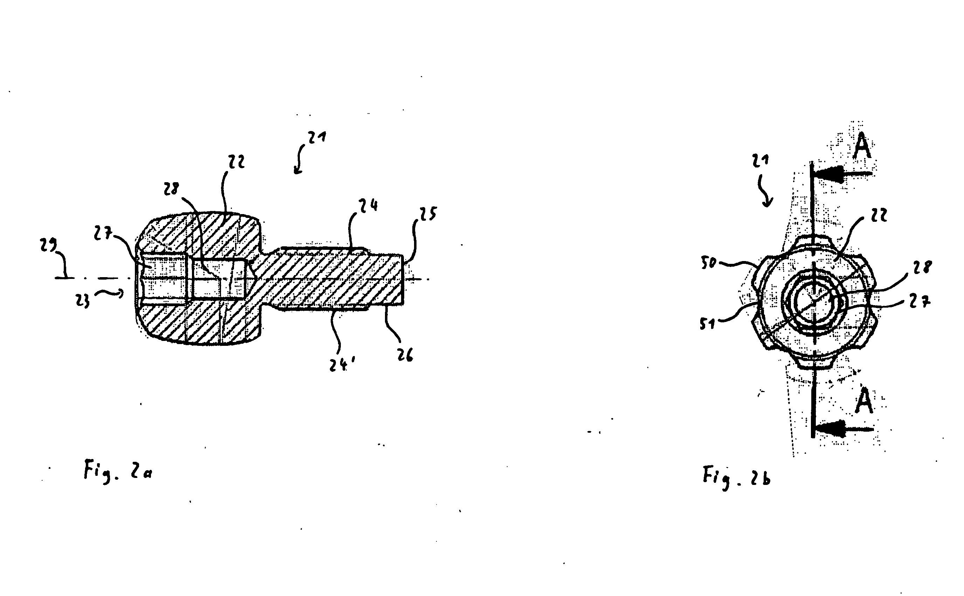

As shown in Fig. 2a in an axial sectional view, a screw 20 comprises a head section with a hand wheel 22, a shaft section with a thread 24' forming a thread section 24, and a tip 25. A cylindrical section 26 is provided between the tip 25 and the thread section 24.

The bore 23 of the screw 21 comprises a first bore 27 and a second bore 28. The first bore 27 is formed adjacent to the surface near the end of the screw opposing the tip 25. Where the first bore 27 intersects the surface of the screw 21, the edges may be inclined or rounded in order to ease insertion of a tool. The first bore has a hexagonal cross-section. The second bore 28 is formed in the bottom of the first bore 27. The second bore 28 has a circular cross-section, the diameter of which is smaller than a diameter of the first bore 27. Both bores 27, 28 are co-axial with the screw axis 29.

As shown in Fig. 2b in a an axial view, the circumferential surface of the hand wheel 22 of the screw 21 exhibits ridges or elevations 50 and grooves 51, in order to improve gripping the hand wheel 22. As also indicated in Fig. 2b, the first bore 27 is larger in diameter than the second bore 28, the first bore 27 having hexagonal and the second bore 28 having circular cross-sections. Fig. 2a is the sectional view along the line denoted "A - A" in Fig. 2b.

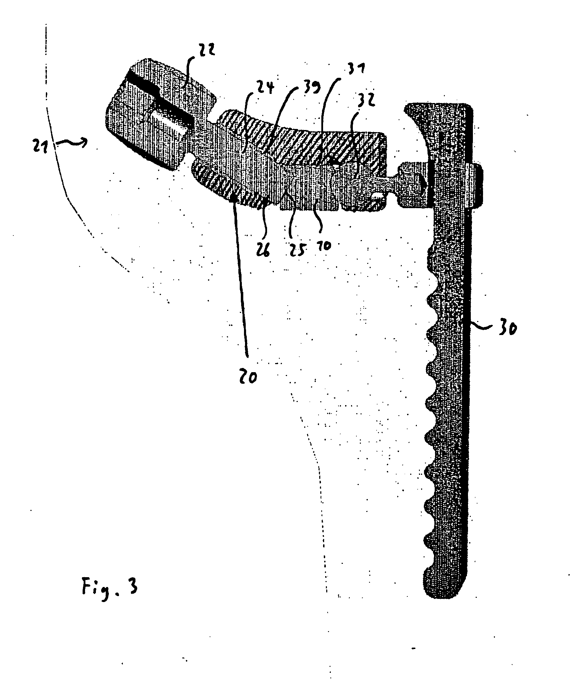

As can be seen in the sectional view of Fig. 3, a screw 21 is inserted into a holding element 20, the screw 21 comprising a head section with a hand wheel 22, a thread section 24, a cylindrical section 26, and a tip 25. The thread section 24 of the screw 21 matches at least partially with a threaded section 39 of the holding element 20. A rod 10 can be held within the recess 31 of the holding element 20 by turning the screw 21 until the tip 25 of the screw protrudes into the recess 31; in this way the holding element 21 can be mounted on the rod 10, but remain movable along the rod 10. When the screw 21 is turned to protrude further into the recess 31 to contact the rod 10 and is tightened, the holding element 20 is clamped on the rod 10 by pressing the rod 10 against the ball 32 of a ball-and-socket joint connecting the blade 30 to the holding element 20. Inversely, when the screw 21 is turned in the other direction, the holding element 20 is loosened so that it can be shifted on the rod 10 and the blade can be rotated. When the screw 21 is further loosened, the holding element 20 can be removed from the rod 10 and the retractor thus disassembled. The axial motion path length of the screw 21 is sufficient for inhibiting or permitting extraction of the rod 10 from the recess 31, as well as for clamping the rod 10 within the holding element 21.

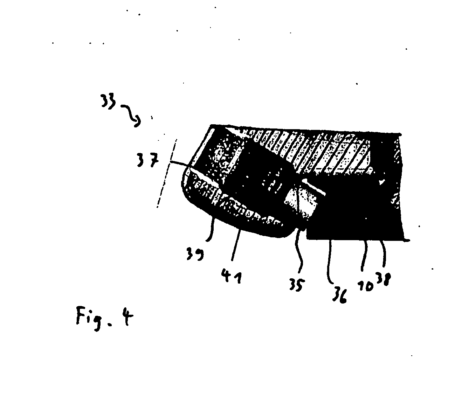

Further functional elements can be mounted and fixed in a similar manner on the rod 10. In particular and as shown in Fig. 4, the light guide holder 41 can be mounted and fixed on the rod 10 by means of a further screw 33 which comprises a thread section 34 to match a threaded section 39 of the functional element, a cylindrical section 35, a tip 36, and an axial bore 37. As explained above in relation to the screw 21, by turning the further screw 33 3 the light guide holder 41 can be held on the rod 10 and can be clamped on the rod 10 by tightening the further screw 33. In particular, when the further screw 33 is tightened, the tip 36 of the screw presses the rod 10 against a protrusion 38 of the light guide holder 41, forming a rigid unit with the rod 10. In total, the further screw 33 may be designed as the screw 21, but without the hand wheel 22.

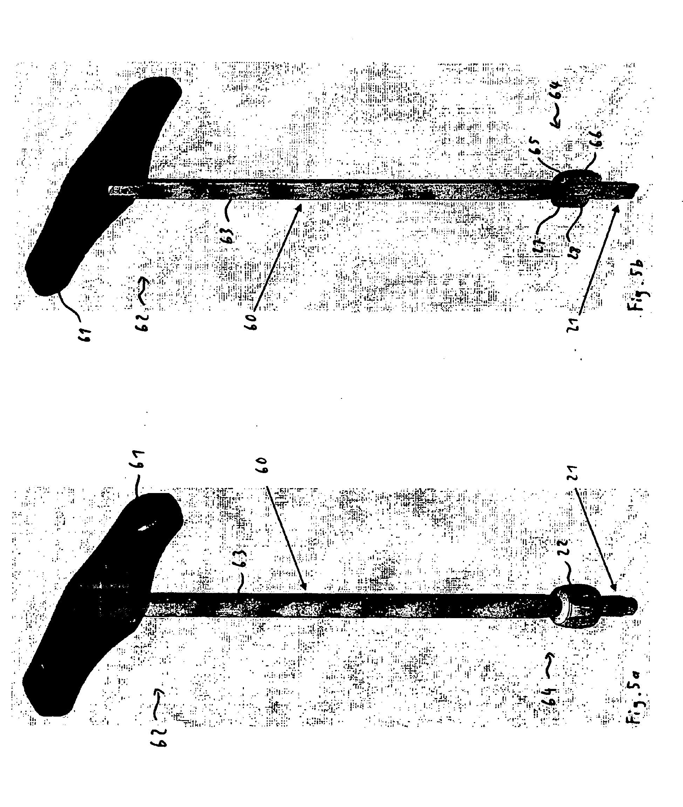

As shown in Figs. 5a and 5b, a screwdriver 60 is configured to match the bore of a screw 21. The screwdriver 60 comprises a handle 61 arranged on a proximal end 62 of an elongate shaft 63. At a distal end 64 of the shaft 63, a second drive section 66 is formed, which may have a hexagonal outer cross-sectional contour matching a hexagonal bore in a further screw for driving the further screw. In the screw 21, the second drive section 66 fits loosely into a second bore 28, which has a circular cross-section with a diameter slightly larger than the largest diameter of the second drive section 66. To the proximal side of the second drive section 66, the shaft 63 comprises a first drive section 65, which may have a hexagonal outer cross-sectional contour matching a hexagonal cross-section of the first bore 27 for driving the screw 21.

Thus, when the screwdriver 60 is inserted into the bores 27, 28 of the screw 21, the second drive section 66 being inserted into the second bore 28 helps to guide the first drive section 65 of the screwdriver 60 into the first bore 27, but itself is not capable of transmitting a torque upon the screw 21. The screw 21 is driven by the hexagonal first drive section 65 matching the hexagonal first bore 28 of the screw 21. If less torque its to be transferred, e. g. for placing the holding element 20 on the rod 10 (see Fig. 3), the screw 21 can be turned by hand by gripping the hand wheel 22.

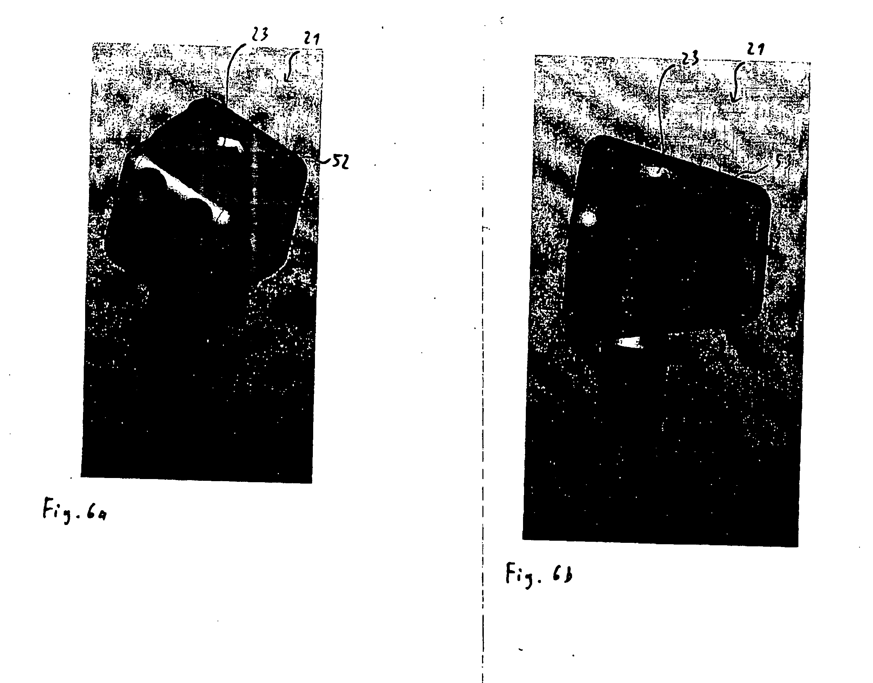

Fig. 6a shows a generally quadratic handle 52 of a screw 21, and Fig. 6b shows a screw 21 with a handle of generally triangular shape. The screw 21 further comprises a bore 23, a thread section 24, a cylindrical section 26, and tip 25, as described above. Screws with various sizes and shapes of handles may be provided for permitting the surgeon to choose the most suitable handle in any particular situation.

### Reference numerals

- 1: Surgical retractor
- 2: Rail
- 3: First arm
- 4: Second arm
- 5: Block
- 6: Bore
- 7: Toothed edge
- 8: Drive shaft
- 10, 10', 10": Rod
- 20, 20', 20", 20"', 20"": Holding element
- 21, 21', 21 ", 21 "', 21"": Screw
- 22, 22', 22", 22"', 22"": Hand wheel
- 23, 23', 23", 23"', 23"": Bore
- 24: Thread section
- 24': Thread
- 25: Tip
- 26: Cylindrical section
- 27: First bore
- 28: Second bore
- 29: Screw axis
- 30, 30', 30", 30"', 30"": Blade
- 31: Recess
- 32: Ball
- 33: Further screw
- 34: Thread section
- 35: Cylindrical section
- 36: Tip
- 37: Bore
- 38: Protrusion
- 39: Threaded section
- 40: Illumination device
- 41: Light guide holder
- 42: Light guide
- 43: Light connector
- 44: Snap-in mechanism
- 50: Elevation
- 51: Groove
- 52: Quadratic handle
- 53: Triangular handle
- 60: Screwdriver
- 61: Handle
- 62: Proximal end
- 63: Shaft
- 64: Distal end
- 65: First drive section
- 66: Second drive section

## Claims

1. Surgical instrument, comprising a first member and a second member, the first member being mountable upon and/or fixable with respect to a second member of the surgical instrument by means of a screw (21, 21', 21 ", 21"', 21 ""), the screw (21, 21', 21 ", 21 "', 21"") having a screw axis (29) and comprising a tip (25) on a first end of the screw, a thread section (24), and a head section adjacent to a second end of the screw, the head section comprising handle means for turning the screw by hand, **characterized in that** the screw further comprises coupling means at the second end for coupling with a drive section (65, 66) of a tool.

2. Surgical instrument according to claim 1, **characterized by** a third member movable with respect to the first and/or second member by means of a drive mechanism, the drive mechanism being operable by turning a shaft of the drive mechanism, the shaft (8) of the drive mechanism comprising a head section adjacent to an end of the shaft, the head section comprising handle means for turning the shaft by hand and coupling means for coupling with the drive section (65, 66) of the tool.

3. Surgical instrument according to claims 1 or 2, **characterized in that** the coupling means comprise a first axial bore (27) and a second axial bore (28) in a bottom of the first axial bore (27).

4. Surgical instrument according to the preceding claim, **characterized in that** the first axial bore (27) has a hexagonal cross-section for coupling with the drive section (65) of a hexagonal screwdriver (60) and the second axial bore (28) has a circular cross-section.

5. Surgical instrument according to claims 3 or 4, **characterized by** a fourth member mountable and/or fixable to the first or second member by a further screw (33), the screw comprising coupling means for engaging a first drive section (65) of the tool in the first axial bore (27), while the further screw (33) comprises coupling means for engaging a second drive section (66) of the tool.

6. Surgical instrument set comprising a surgical instrument according to any one of the preceding claims and a tool with a drive section (65, 66) for coupling with the coupling means of the screw (21, 21', 21 ", 21"', 21"") and a tool handle (61) for operating the tool.

7. Surgical instrument set according to claim 6, **characterized in that** the tool is a surgical screwdriver (60).

8. Surgical instrument set according to claim 7, **characterized in that** the screwdriver (60) comprises a first drive section (65) with a hexagonal cross-section and a second drive section (66) with a hexagonal cross-section, the first drive section having a larger diameter than the second drive section and the second drive section being situated adjacent to the first drive section and adjacent to a distal end (64) of the screwdriver (60).

## Patentansprüche

1. Chirurgisches Instrument, umfassend ein erstes Element und ein zweites Element, wobei das erste Element an einem zweiten Element des chirurgischen Instruments anhand einer Schraube (21, 21', 21", 21'"', 21"") montierbar und/oder im Verhältnis dazu fixierbar ist, wobei die Schraube (21, 21', 21", 21"', 21"") eine Schraubenachse (29) aufweist und eine Spitze (25) an einem ersten Ende der Schraube, einen Gewindeabschnitt (24) und einen Kopfabschnitt neben einem zweiten Ende der Schraube umfasst, wobei der Kopfabschnitt Griffmittel umfasst, um die Schraube per Hand zu drehen, **dadurch gekennzeichnet, dass** die Schraube ferner Koppelmittel an dem zweiten Ende zum Koppeln mit einem Eintriebabschnitt (65, 66) eines Werkzeugs umfasst.

2. Chirurgisches Instrument nach Anspruch 1, **gekennzeichnet durch** ein drittes Element, das im Verhältnis zu dem ersten und/oder zweiten Element anhand eines Eintriebmechanismus bewegbar ist, wobei der Eintriebmechanismus betätigt werden kann, indem man einen Schaft des Eintriebmechanismus dreht, wobei der Schaft (8) des Eintriebmechanismus einen Kopfabschnitt neben einem Ende des Schafts umfasst, wobei der Kopfabschnitt Griffmittel zum Drehen des Schafts per Hand und Kopplungsmittel zum Koppeln mit dem Eintriebabschnitt (65, 66) des Werkzeugs umfasst.

3. Chirurgisches Instrument nach Anspruch 1 oder 2, **dadurch gekennzeichnet, dass** die Kopplungsmittel eine erste Axialbohrung (27) und eine zweite Axialbohrung (28) in einem Boden der ersten Axialbohrung (27) umfassen.

4. Chirurgisches Instrument nach dem vorhergehenden Anspruch, **dadurch gekennzeichnet, dass** die erste Axialbohrung (27) einen hexagonalen Querschnitt zum Koppeln mit dem Eintriebabschnitt (65) eines hexagonalen Schraubendrehers (60) und die zweite Axialbohrung (28) einen kreisförmigen Querschnitt aufweist.

5. Chirurgisches Instrument nach Anspruch 3 oder 4, **gekennzeichnet durch** ein viertes Element, das an dem ersten oder zweiten Element **durch** eine weitere Schraube (33) montierbar und/oder fixierbar ist, wobei die Schraube Kopplungsmittel umfasst, um einen ersten Eintriebabschnitt (65) des Werkzeugs mit der ersten Axialbohrung (27) in Eingriff zu bringen, während die weitere Schraube (33) Kopplungsmittel umfasst, um einen zweiten Eintriebabschnitt (66) des Werkzeugs in Eingriff zu bringen.

6. Chirurgischer Instrumentensatz, umfassend ein chirurgisches Instrument nach einem der vorhergehenden Ansprüche und ein Werkzeug mit einem Eintriebabschnitt (65, 66) zum Koppeln mit den Kopplungsmitteln der Schraube (21, 21', 21", 21"', 21"") und einen Werkzeuggriff (61) zum Betätigen des Werkzeugs.

7. Chirurgischer Instrumentensatz nach Anspruch 6, **dadurch gekennzeichnet, dass** das Werkzeug ein chirurgischer Schraubendreher (60) ist.

8. Chirurgischer Instrumentensatz nach Anspruch 7, **dadurch gekennzeichnet, dass** der Schraubendreher (60) einen ersten Eintriebabschnitt (65) mit einem hexagonalen Querschnitt und einen zweiten Eintriebabschnitt (66) mit einem hexagonalen Querschnitt umfasst, wobei der erste Eintriebabschnitt einen größeren Durchmesser als der zweite Eintriebabschnitt aufweist und sich der zweite Eintriebabschnitt neben dem ersten Eintriebabschnitt und neben einem distalen Ende (64) des Schraubendrehers (60) befindet.

## Revendications

1. Instrument chirurgical, comportant un premier organe et un second organe, le premier organe pouvant être monté sur un second organe de l'instrument chirurgical et/ou fixé par rapport à ce dernier au moyen d'une vis (21, 21', 21", 21"', 21""), la vis (21, 21', 21", 21"', 21"") présentant un axe (29) de vis et comportant une pointe (25) sur une première extrémité de la vis, une section (24) filetée, et une section de tête adjacente à une seconde extrémité de la vis, la section de tête comportant un moyen formant poignée pour tourner la vis à la main, **caractérisé en ce que** la vis comporte en outre un moyen d'accouplement au niveau de la seconde extrémité pour s'accoupler avec une section (65, 66) d'entraînement d'un outil.

2. Instrument chirurgical selon la revendication 1, **caractérisé par** un troisième organe qui peut être déplacé par rapport au premier et/ou au second organe au moyen d'un mécanisme d'entraînement, le mécanisme d'entraînement pouvant fonctionner en faisant tourner un arbre du mécanisme d'entraînement, l'arbre (8) du mécanisme d'entraînement comportant une section de tête adjacente à une extrémité de l'arbre, la section de tête comportant un moyen formant poignée pour faire tourner l'arbre à la main et un moyen d'accouplement pour s'accoupler avec la section (65, 66) d'entraînement de l'outil.

3. Instrument chirurgical selon la revendication 1 ou 2, **caractérisé en ce que** le moyen d'accouplement comporte un premier alésage (27) axial et un second alésage (28) axial dans un fond du premier alésage (27) axial.

4. Instrument chirurgical selon la revendication précédente, **caractérisé en ce que** le premier alésage (27) axial présente une section transversale hexagonale pour s'accoupler avec la section (65) d'entraînement d'un tournevis (60) hexagonal et le second alésage (28) axial présente une section transversale circulaire.

5. Instrument chirurgical selon la revendication 3 ou 4, **caractérisé par** un quatrième organe pouvant être monté et/ou fixé sur le premier ou le second organe par une autre vis (33), la vis comportant un moyen d'accouplement pour mettre en prise une première section (65) d'entraînement de l'outil dans le premier alésage (27) axial, tandis que l'autre vis (33) comporte un moyen d'accouplement pour mettre en prise une seconde section (66) d'entraînement de l'outil.

6. Ensemble d'instruments chirurgicaux comportant un instrument chirurgical selon l'une quelconque des revendications précédentes et un outil présentant une section (65, 66) d'entraînement pour s'accoupler au moyen d'accouplement de la vis (21, 21', 21", 21"', 21"") et une poignée (61) d'outil pour faire fonctionner l'outil.

7. Ensemble d'instruments chirurgicaux selon la revendication 6, **caractérisé en ce que** l'outil est un tournevis (60) chirurgical.

8. Ensemble d'instruments chirurgicaux selon la revendication 7, **caractérisé en ce que** le tournevis (60) comporte une première section (65) d'entraînement présentant une section transversale hexagonale et une seconde section (66) d'entraînement présentant une section transversale hexagonale, la première section d'entraînement ayant un diamètre plus grand que la seconde section d'entraînement et la seconde section d'entraînement étant située de manière adjacente à la première section d'entraînement et de manière adjacente à une extrémité (64) distale du tournevis (60).
